# EUROPÄISCHE PATENTSCHRIFT

(11) **EP 1 230 339 B1**
(45) Veröffentlichungstag und Bekanntmachung des Hinweises auf die Patenterteilung: **21.01.2004**
(21) Anmeldenummer: 00983142.1
(22) Anmeldetag: 16.11.2000
(51) Int. Cl.: C12M 1/107

(54) **VORRICHTUNG UND VERFAHREN ZUM GÄREN ORGANISCHER SUBSTANZEN**
DEVICE AND METHOD FOR FERMENTING ORGANIC SUBSTANCES
DISPOSITIF ET PROCEDE DE FERMENTATION DE SUBSTANCES ORGANIQUES

(30) Priorität: 16.11.1999 DE 19954903
(43) Veröffentlichungstag der Anmeldung: 14.08.2002
(73) Patentinhaber: Lipp, Xaver, 73479 Ellwangen (DE)
(72) Erfinder: Lipp, Xaver, 73479 Ellwangen (DE)
(74) Vertreter: Patentanwälte Bartels und Partner
(86) Internationale Anmeldenummer: PCT/EP2000/011366
(87) Internationale Veröffentlichungsnummer: WO 2001/036584

(56) Entgegenhaltungen:
- EP-A- 0 167 696
- WO-A-83/03884
- DE-A- 4 120 988
- DE-B- 2 946 884
- DE-C- 19 714 342
- FR-A- 2 461 004
- US-A- 4 090 940

## Beschreibung

Die Erfindung betrifft eine Vorrichtung und ein Verfahren zum Gären organischer Substanzen.

Die aus der DE 197 56 485 A1 bekannte Vorrichtung weist einen die organischen Substanzen bis zu einem Füllstand aufnehmenden Gärraum und eine Mischeinrichtung in Form eines Rührwerks auf. Das Rührwerk saugt über ein Rührrohr oder einen Rührschacht die organischen Substanzen aus einem Bereich nahe des Füllstandes ab und sorgt auf diese Weise für eine gute Durchmischung der zu vergärenden bzw. faulenden Substanzen.

Insbesondere beim Einsatz landwirtschaftlicher Produkte und Abfallprodukte, beispielsweise Stroh oder Heu, bilden sich an der Oberfläche der organischen Substanzen verhältnismäßig dicke und zähe Schwimm- und Deckschichten aus. Diese behindern das Aufsteigen und Austreten von Faulgas und damit den Gär- bzw. Faulprozeß.

Die DE 32 26 698 A1 und die EP 0 374 708 A1 zeigen eine Vorrichtung zur Erzeugung von Biogas mit einer in der Horizontalen liegenden Längsachse, wobei die Reaktionstrommel um eine waagrechte Achse drehbar gelagert und von einem Elektromotor angetrieben ist. In der Reaktionstrommel ist eine feststehende Förderschnecke angeordnet, die nahe ihrer mit der Längsachse der Vorrichtung zusammenfallenden Achse kreisförmige Durchbrüche für den axialen Durchtritt von Substrat und Biogas aufweist.

Die DE 32 39 304 A1 zeigt einen mit horizontal ausgerichteter Längsachse zylindrischen Biogasreaktor, der ein mit Zinken versehenes Rührwerk zur Durchmischung der in den Biogasreaktor eingebrachten Gülle aufweist.

Die DE 33 09 356 A1 zeigt ein Verfahren zur Gewinnung von Biogas und eine Vorrichtung zur Durchführung dieses Verfahrens, bei der in einem zylindrischen Reaktor mit horizontaler Längsachse ein Rührwerk vorgesehen ist, das an seinen Rührflügeln eine Schöpfvorrichtung in Form eines Hohlkörpers aufweist, der sich beim Durchstreichen des Gasraumes mit Gas füllt und beim anschließenden Eintauchen in den Flüssigkeitsraum das Gas in dem Flüssigkeitsraum entweichen lässt. Dadurch soll das bereits aufgestiegene Biogas dem Reaktionsgut wieder zugeführt werden.
Die Längsachse des Reaktors fällt mit der Drehachse des Rührwerks zusammen.

Die DE 37 37 870 A1 zeigt ein Verfahren und eine Vorrichtung zur Herstellung von Biogas mit einem zylindrischen Gärraum mit vertikal ausgerichteter Längsachse, in dem zur Durchmischung der organischen Substanzen zwei parallel zur Behälterachse axial vertikal bewegbare Mischkörper aus Beton vorgesehen sind, die innerhalb der Flüssigkeit auf und ab bewegbar sind.

Die DE 295 20 618 U1 zeigt einen Fermenter mit einem liegenden Tank, der ein Fördersystem aufweist, das im Bereich des Bodens des Tanks eine von einem Auslaß weggerichtete gegenläufige Förderbewegung verursacht, so daß auf den Boden abgesunkene Reststoffe von dem Auslaß in einen vom Auslaß entfernten Teil des Fermenterraums rückgefördert werden.

Die DE 30 27 085 A1 zeigt einen liegend angeordneten zylindrischen Biogasreaktor mit einer zur Aufrechterhaltung der Reaktionstemperatur vorgesehenen Heizeinrichtung, die einen Wärmespeicher umfasst, in den mittels eines Wärmetauschers in dem auszutragenden Gut enthaltene Wärme einkoppelbar ist.

Die DE 44 16 521 A1 zeigt einen zylindrischen Fermenter mit horizontal ausgerichteter Längsachse und einem um die horizontale Längsachse drehbaren Rührwerk. Das Rührwerk dient dazu, sedimentierte Feststoffe vom Boden des Reaktorraums aufzuschaufeln, damit diese im Pfropfstrom des Reaktors weiter transportiert werden können und nicht zu Verstopfungen führen.

Die DE 196 17 734 C1 zeigt ein Verfahren und eine Vorrichtung zur Herstellung eines gärfähigen Gemisches aus biologischen Abfällen, die gesiebt, zerkleinert, von steinernen und metallischen Abfällen getrennt und anschließend als Suspension einem Gärbehälier zugeführt werden.

Die WO 94/21565 zeigt eine Vorrichtung zum kontinuierlichen Kochen von organischen Substanzen in einem zylindrischen Reaktor mit horizontal verlaufender Längsachse und einer in dem Reaktor angeordneten Rotationseinrichtung, deren Rotationsachse mit der Längsachse des Reaktors zusammenfällt. Die Rotationseinrichtung teilt den Reaktorraum in mindestens drei Abschnitte, wobei jeder Abschnitt durch zwei einander gegenüberliegenden Platten der Rotationseinrichtung begrenzt ist.

Die DE 36 44 120 A1 zeigt einen Elektromixer zum Umströmen und Homogenisieren von Gülle und anderen Flüssigkeiten. Der zugehörige Elektromotor kann aus der umzuwälzenden Flüssigkeit insbesondere zu Wartungs- und Reparaturarbeiten mittels eines an dem Güllebecken angebrachten Tragkranes hervorgehoben werden. Im Betrieb ist der Elektromixer und insbesondere der Mixerpropeller nahe dem Boden des Güllebeckens angeordnet zum Umwälzen der Gülle und insbesondere zum Aufmischen der sich am Boden absetzenden Schicht.

Die US 4 090 940 zeigt eine Vorrichtung, bei der Mischmittel an einem endlos umlaufenden Förderband angebracht ist.

Die DE 41 20 988 A1 zeigt eine gasdichte Wellen- bzw. Schaftdurchführung für eine Rührwerksanordnung, wobei ein einziger Rührpropeller in den Gärraum hineinschwenkbar ist.

Die FR 2 461 004 A1 zeigt eine Vorrichtung mit einer vertikal ausgerichteten und unterhalb des füllstandes angeordneten Förderschnecke, die von einem hydraulischen Motor antreibbar ist.

Die EP 0 167 696 A1 zeigt eine Vorrichtung mit einer vertikal in dem Gärbehälter ausgerichteten Rührachse, an der kappenartige und vorzugsweise schirmförmige Einbaukörper als Mischmittel angeordnet sind.

Die WO 83/038 84 zeigt eine flexible Abdeckung für einen Gasspeicher einer gattungsgemäßen Vorrichtung.

Die DE 197 14 342 C1 zeigt eine Vorrichtung mit einem einzigen in den Füllstand eintauchbaren und an einem Tauchmotor angeordneten Rührpropeller.

Die DE 29 46 884 B zeigt eine Vorrichtung entsprechend dem Oberbegriff des Anspruchs 1. Die Mischmittel bzw. Rührschaufeln usw. sind dabei an einem endlos umlaufenden und im wesentlichen vertikal ausgerichteten Förderband angeordnet, das bis nahe an den Boden des Gärraums reicht.

Der Erfindung liegt das Problem zugrunde, eine Vorrichtung und ein Verfahren zum Gären organischer Substanzen bereitzustellen, welche die Nachteile des Standes der Technik überwinden. Insbesondere soll die Gasabscheidung erhöht sein und der Gärprozeß beschleunigt werden. Die Vorrichtung soll dabei einfach und kostengünstig aufgebaut sein und das Verfahren einfach und zuverlässig sein, insbesondere bei Bedarf automatisierbar sein.

Das Problem ist durch die im Anspruch 1 bestimmte Vorrichtung und durch das im nebengeordneten Anspruch bestimmte Verfahren gelöst. Besondere Ausführungsarten der Erfindung sind in den Unteransprüchen bestimmt.

Dadurch, daß die Mischmittel der Mischeinrichtung mindestens teilweise über den Füllstand hinausragend aus den organischen Substanzen heraus und anschließend wieder mindestens teilweise in die organischen Substanzen eintauchend bewegbar sind, wird eine Schwimm- bzw. Deckschicht zuverlässig und nachhaltig aufgerissen, zerstört und/oder aufgelöst. Durch das Aufreißen der Deckschicht können die sich bildenden Faulgase ungehindert aus den Substanzen austreten. Außerdem ist durch die Mischeinrichtung die Durchmischung der Substanzen verbessert und damit die Gasabscheideaktivität erhöht. Die Mischmittel ragen vorzugsweise nur soweit über den Füllstand hinaus, daß sie beim anschließenden Eintauchen in die organischen Substanzen die Deckschicht in ausreichendem Maße aufreißen bzw. zerstören.

Die Mischmittel sind auf einer Welle angeordnet und um eine Achse rotierend antreibbar. Die Welle ist im wesentlichen horizontal ausgerichtet, schließt mit der Längsachse des Faulbehälters vorzugsweise im wesentlichen einen rechten Winkel ein, und ist vorzugsweise unterhalb des Füllstandes, aber oberhalb der halben Höhe des Gärraumes und insbesondere nahe dem Füllstand angeordnet. Die Welle verläuft in diesem Fall in dem mit organischen Substanzen angefüllten Gärraum. Sofern an einer Welle mehrere Mischmittel angeordnet sind, sind diese vorzugsweise so verteilt und ausgerichtet, daß der Rotation in jeder Stellung der Welle annähernd der gleiche Widerstand von den organischen Substanzen bzw. der zu zerstörenden Deckschicht entgegengebracht wird. Insbesondere befinden sich in diesem Fall jeweils eine oder mehrere der Mischmittel in vollständigem Eingriff mit den organischen Substanzen bzw. der Deckschicht und ein im wesentlichen gleichbleibender Teil der Mischmittel ragt über den Füllstand hinaus. Der Antrieb der Achse erfolgt vorzugsweise von außerhalb der Vorrichtung, entweder unmittelbar über eine durch die Vorrichtungswand hindurchtretende Antriebswelle oder mittelbar über eine mindestens zum Teil außerhalb der organischen Substanzen angeordneten und von außerhalb der Vorrichtung antreibbaren Getriebeeinrichtung. Sofern mehrere Mischmittel entlang der Achse vorzugsweise äquidistant hintereinander in einer Schraubenlinie angeordnet sind, ergibt sich in einfacher Weise eine nahezu gleichmäßige Belastung des Antriebs der Mischeinrichtung. Darüber hinaus können bei einer derartigen Anordnung die organischen Substanzen beim Betrieb der Mischeinrichtung in eine Dreh-/Rührbewegung versetzt werden, und insbesondere auch parallel zur Achse in Bewegung versetzt werden, wodurch die Durchmischung der Substanzen weiter verbessert ist. Auch eine Anordnung der Mischmittel vorzugsweise äquidistant hintereinander alternierend diametral zur Achse ist möglich.

Wenn die Mischmittel und die Welle in ihrer Höhe in Bezug auf den Füllstand einstellbar ist, ist die Position der Mischeinrichtung an unterschiedliche Füllstände und/oder Dicken der sich gebildeten Deckschicht anpaßbar. Dadurch ist die Auflösung der Deckschicht weiter verbessert. Die Einstellbarkeit kann insbesondere dann einfach realisiert werden, wenn die Antriebswelle beispielsweise über einen Zahnrad-, Riemen- oder Kettenantrieb von einem zumindest teilweise innerhalb der Vorrichtung angeordneten Getriebeeinrichtung antreibbar ist oder wenn die Antriebswelle oberhalb des Füllstandes angeordnet ist.
Die Höheneinstellung kann mittels eines Seil- oder Kettenzuges erfolgen. Die Lager der Antriebswelle können zu diesem Zweck in Führungsschienen vertikal verschiebbar sein. Alternativ oder ergänzend zur vertikalen Einstellbarkeit kann die Antriebswelle auch horizontal parallel verschiebbar oder schwenkbar und vertikal schwenkbar sein.

Dadurch, daß die Mischwinkel flächig ausgebildet sind, ist deren Effektivität bei der Auflösung der Deckschicht und bei der Durchmischung der Substanzen erhöht. Die Mischmittel können dabei die Form eines Paddelblatts aufweisen, wobei die von dem Paddelblatt ausgebildete Fläche im wesentlichen eben ist und parallel zu der Achse ausgerichtet ist. Beim Eintauchen derartiger Mischmittel werden verhältnismäßig großflächige Stücke der Deckschicht in die flüssigen organischen Substanzen hineingedrückt und damit entsprechend große Löcher in die Deckschicht gerissen. Bei der Ausgestaltung der Mischmittel in Form eines schräg gestellten Propellerblatts, bei dem die von dem Propellerblatt ausgebildete Fläche mindestens zum Teil mit der Achse einen spitzen Winkel einschließt, werden die organischen Substanzen bzw. die Deckschicht beim Eintauchen des Propellerblatts auch parallel zur Achse in Bewegung gesetzt. Außerdem ist bei derartigen Mischmitteln die Belastung des Antriebssystems der Mischeinrichtung gleichförmiger, insbesondere treten geringere Lastwechsel beim Eintauchen und Austreten der Mischmittel in bzw. aus den organischen Substanzen auf.

Beispielsweise über die Oberfläche verteilt kann die Vorrichtung auch mehrere Mischeinrichtungen mit insbesondere mehreren Achsen bzw. Antriebswellen aufweisen, die nebeneinander und vorzugsweise parallel zueinander angeordnet sind. Die mehreren Mischeinrichtungen können einzeln oder gemeinsam von einer oder mehreren Antriebseinrichtungen antreibbar sein. Die einzelnen Mischeinrichtungen können insbesondere hinsichtlich ihrer Mischmittel identisch aufgebaut sein und sich beispielsweise lediglich in ihrer axialen Erstreckung unterscheiden, oder insbesondere hinsichtlich ihrer Mischmittel unterschiedlich aufgebaut sein, insbesondere kann die mittlere Mischeinrichtung mit großflächigeren Mischmitteln ausgestattet sein als die randnahen Mischeinrichtungen.

Wenn die Vorrichtung weiterhin eine Schichtdickenmeßeinrichtung aufweist, zur Bestimmung der Dicke einer an der Oberfläche der organischen Substanzen schwimmenden Deckschicht, kann die Mischeinrichtung nicht nur entsprechend gewonnenen Erfahrungswerten in Betrieb genommen werden, sondern immer dann beispielsweise von einer Steuereinrichtung automatisch eingeschaltet werden, wenn die Deckschicht eine kritische Dicke erreicht. In entsprechender Weise ist die Mischeinrichtung aufgrund des Meßergebnisses ausschaltbar, sobald die Deckschicht in ausreichendem Maße aufgelöst ist. Zur Schichtdickenmessung eignen sich neben schwimmerbasierten elektromechanischen Geräten, bei denen beispielsweise der Abstand zwischen einem ersten Schwimmer, der an der Oberfläche der organischen Substanzen schwimmt, und einem zweiten Schwimmer, der an der Oberfläche der Deckschicht schwimmt, bestimmbar ist, auch andere zur Verfügung stehende Meßverfahren, beispielsweise beruhend auf Ultraschall-, optischen oder magnetischen Meßverfahren.

Mit einem Gasraum, der insbesondere durch eine in die Vorrichtung integrierte und eine aufblähbare Hülle aufweisende Gashaube gebildet ist, kann der beim Gären entstehende gasförmige Energieträger aufgefangen werden. Dieser kann in einem Gaslager zwischengespeichert werden oder unmittelbar einer Einrichtung zum Wandeln in elektrische und/oder thermische Energie zugeführt werden. Auf diese Weise kann die Vorrichtung energetisch autark betrieben werden und eventuell überschüssiges Gas kann weiteren Verbrauchern zur Verfügung gestellt werden.

Die Erfindung ist auch durch ein Verfahren zum Gären organischer Substanzen in einer erfindungsgemäßen Vorrichtung gelöst, bei dem die Mischeinrichtung mindestens dann betrieben wird, wenn sich an der Oberfläche der organischen Substanzen im Gärraum eine den Aufstieg von Gasblasen behinderte Deckschicht gebildet hat. Dadurch, daß die Gasblasen nicht frei aufsteigen können, ist auch der Gär- bzw. Faulprozeß behindert oder gehemmt. Die Behinderung des Gasaufstiegs und damit der erforderliche Zeitpunkt zum Betreiben der Mischeinrichtung kann auf unterschiedliche Weise ermittelt werden, beispielsweise aufgrund gewonnener Erfahrungswerte, gegebenenfalls unter Berücksichtigung der Prozeßbedingungen wie beispielsweise Temperatur und/oder Art und Zusammensetzung der eingefüllten organischen Substanzen, Messung der Gasabscheidung, Messung des Temperaturverlaufs oder Messung der Dicke der sich bildenden Deckschicht. Darüber hinaus kann die Mischeinrichtung in vorgebbaren regelmäßigen Zeitabständen betrieben werden, die im wesentlichen von den Prozeßbedingungen abhängig sind.

Weitere Vorteile, Merkmale und Einzelheiten der Erfindung ergeben sich aus den Unteransprüchen sowie der nachfolgenden Beschreibung, in der unter Bezugnahme auf die Zeichnungen mehrere Ausführungsbeispiele im einzelnen beschrieben sind. Dabei können die in den Ansprüchen und in der Beschreibung erwähnten Merkmale jeweils einzeln für sich oder in beliebiger Kombination erfindungswesentlich sein.
- Fig. 1: zeigt eine Seitenansicht einer erfindungsgemäßen Vorrichtung im Querschnitt, und
- Fig. 2: zeigt eine Aufsicht auf ein zweites Ausführungsbeispiel der Erfindung.

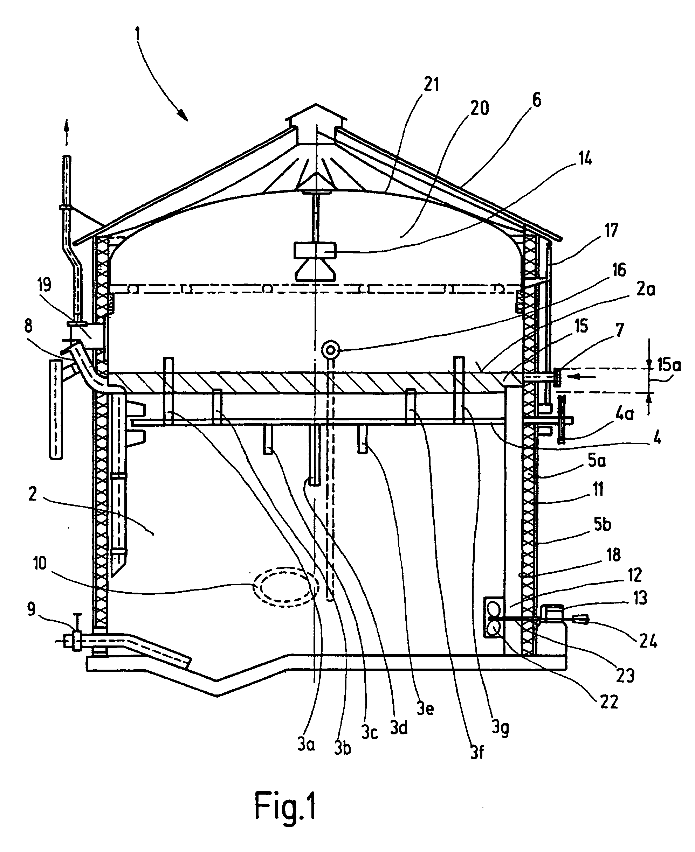

Die Fig. 1 zeigt eine Seitenansicht der erfindungsgemäßen Vorrichtung. Im dargestellten Beispiel bildet die Vorrichtung einen Gär- bzw. Faulbehälter 1 mit einem die organischen Substanzen bis zu einem Füllstand 2a aufnehmenden Gärraum 2, und einer Mischeinrichtung, die antreibbare Mischmittel 3a bis 3g aufweist. Die Mischmittel 3a bis 3g sind mindestens teilweise über den Füllstand 2a hinausragend aus dem organischen Substanzen heraus und anschließend wieder mindestens teilweise in die organischen Substanzen eintauchend bewegbar. Die Mischmittel 3a bis 3g sind um eine Achse mittels einer Welle 4 rotierend antreibbar, wobei die Welle 4 im wesentlichen horizontal ausgerichtet und unterhalb des Füllstandes 2a angeordnet ist. Vorzugsweise ist die Welle 4 auch unterhalb des unteren Rands der auf der Oberfläche der organischen Substanzen schwimmenden Deckschicht 15 angeordnet. Die Welle 4 ist jeweils nahe dem Rand des kreiszylindrischen Faulbehälters 1 gelagert und tritt einseitig durch die Wandung des Faulbehälters 1 hindurch und ist von außerhalb über eine Riemenscheibe 4a antreibbar.

Auf der Welle 4 sind äquidistant hintereinander insgesamt sieben Mischmittel 3a bis 3g in einer Schraubenlinie angeordnet. In umfänglicher Rotationsrichtung sind die Mischmittel 3a bis 3g jeweils um 60° gegeneinander versetzt. In der dargestellten Stellung ragen die äußeren Mischmittel 3a, 3g mit einem Teil ihrer Fläche über den Füllstand 2a hinaus. Die daneben angeordneten Mischmittel 3b, 3f greifen mit einem Teil ihrer Fläche in die Deckschicht 15 ein. Die dazwischenliegenden Mischmittel 3c bis 3e befinden sich in der dargestellten Stellung im Bereich der organischen Substanzen, die im wesentlichen aus einer wässrigen Aufschwemmung von Fäkalien und/oder landwirtschaftlichen Abfallprodukten wie Heu oder Stroh bestehen. Die Mischmittel 3a bis 3g weisen jeweils die Form eines Paddelblatts auf, dessen Fläche im wesentlichen eben ist und parallel zu der Welle 4 ausgerichtet ist. Im Gasraum 20 oberhalb des Gärraums 2 ist eine Ultraschall-Schichtdickenmeßeinrichtung 14 zur Bestimmung der Dicke 15a der Deckschicht 15 angeordnet.

Die den Gärraum 2 begrenzende Wandung weist eine Heizeinrichtung 5a, eine Wärmeisolation 5b und eine im wesentlichen die Tragfähigkeit bereitstellende Verkleidung 11 auf. Über einen Füllstutzen 7 sind frische organische Substanzen dem Gärraum zuführbar. Der Ablauf 8 weist einen integrierten Notablauf auf. Über dein absperrbaren Anschluß 9 erfolgt die Entleerung des Faulbehälters 1. Der Gärraum ist über ein Mannloch 10 von außen zugänglich. Ein Rührwerk 22 befindet sich in einem im wesentlichen horizontalen Abschnitt eines darüber hinaus im wesentlichen vertikal angeordneten Rührrohres 12, das am Rand des Gärraums 2 angeordnet ist. Das Rührwerk 22 ist mittels der Rührwelle 23 von außerhalb des Faulbehälters 1 antreibbar, beispielsweise mittels des Elektromotors 13 oder mittels einer über die Anschlußkupplung 24 anschließbaren Antriebswelle eines Verbrennungsmotors, beispielsweise eines Traktors. Der Faulbehälter 1 weist weiterhin einen Gasraum 20 auf, der insbesondere durch eine in den Faulbehälter 1 integrierte und eine aufblähbare Hülle 21 aufweisende Gashaube gebildet ist, und in dem ein beim Faulen der organischen Substanzen entstehender gasförmiger Energieträger auffangbar ist. Ein Aufsatz, der ein die Hülle 21 vor Witterungseinflüssen schützendes Dach 6 aufweist, ist an der Wandung lösbar befestigt. Der Faulbehälter 1 weist darüber hinaus einen Anschluß 16 zur Gasentnahme und eine Gasinhaltsanzeige 17 auf. Weiterhin ist an der dem Gärraum 2 zugewandten Seite der Wandung eine Temperaturmeßeinrichtung 18 zur Messung der Temperatur der organischen Substanzen vorgesehen. Eine Über- und Unterdrucksicherung 19 schützt den Faulbehälter 1 und insbesondere die Hülle 21 vor einer mechanischen Überbeanspruchung.

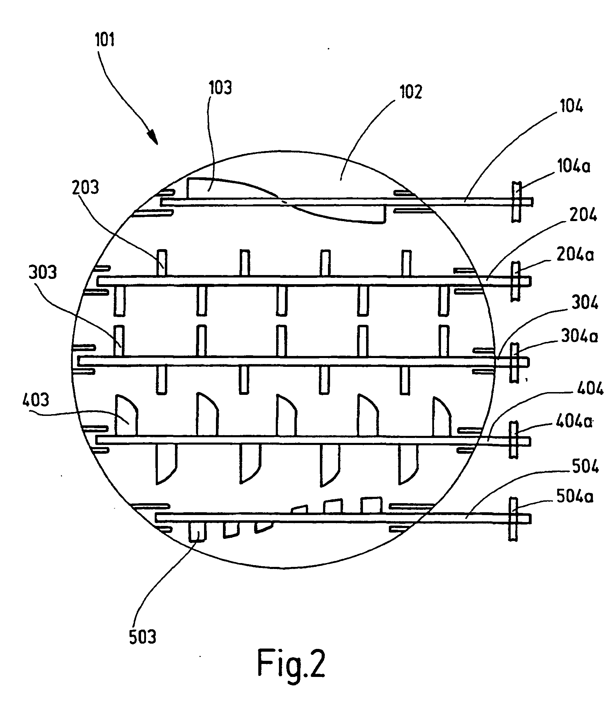

Die Fig. 2 zeigt eine Aufsicht auf eine alternative Ausführungsform der Erfindung. Der Faulbehälter 101 weist mehrere Mischeinrichtungen mit insbesondere mehreren Antriebswellen 104 bis 504 auf, die nebeneinander und parallel zueinander angeordnet sind. Das Mischmittel der ersten randseitigen Mischeinrichtung ist durch einen durch eine halbe Windung um die Welle 104 gedrehten Steg 103 gebildet, dessen Enden jeweils parallel zur Achse der Welle 104 ausgerichtet sind.

Eine in einem ersten Zwischenraum angeordnete erste Zwischenwelle 204 trägt paddelblattförmige Mischmittel 203, die entlang der Welle 204 äquidistant hintereinander alternierend diametral angeordnet sind. In entsprechender Weise sind die Mischmittel 303 ausgestaltet und auf der zentrischen Welle 304 angeordnet. Die Mischmittel 203, 303 sind an einander gegenüberliegenden Stellen entlang der Wellen 204, 304 angeordnet. Je nach Winkellage der Mischmittel 203 in Bezug auf die Winkellage der Mischmittel 303 und in Abhängigkeit der Drehrichtung der Welle 204 in Bezug auf die Drehrichtung der Welle 304 kann die Deckschicht 15 in mehr oder weniger große Einzelstücke aufgerissen werden und diese Einzelstücke zusammen mit den organischen Substanzen alternativ oder ergänzend zu der vom Rührwerk 22 verursachten Dreh/Rührbewegung in Bewegung versetzt werden.

Die Mischmittel 403 der in einem zweiten Zwischenraum angeordneten zweiten Zwischenwelle 404 weisen jeweils die Form eines schräggestellten Propellerblatts auf. Sie sind axial an Zwischenpositionen in Bezug auf die Mischmittel 303 der benachbarten zentrischen Welle 304 angeordnet. Dadurch kann bei entsprechender Winkellage der Wellen 304 und 404 und bei übereinstimmenden Rotationsgeschwindigkeiten die radiale Länge der Mischmittel 303, 403 größer als der halbe Abstand zwischen den Wellen 304, 404 gewählt werden.

Die Mischmittel 503 der zweiten randseitigen Welle 504 sind als Stegabschnitte ausgebildet, die entlang einer einen Winkelbereich von 180° überstreichenden Schraubenlinie angeordnet sind.

Jede Antriebswelle 104 bis 504 weist an ihrem außerhalb des Gärraums 102 befindlichen Ende eine Riemenscheibe 104a bis 504a auf, über die sie antreibbar ist. Der Antrieb kann dabei über einen gemeinsam über alle Riemenscheiben 104a bis 504a geführten Antriebsriemen erfolgen oder einzeln über individuelle Antriebsriemen oder einen einzigen Antriebsriemen, der wahlweise über eine oder mehrere der Riemenscheiben 104a bis 504a führbar ist. Anstelle des Antriebsriemens können andere Antriebsmittel eingesetzt werden, beispielsweise ein Zahnrad- oder Kettenantrieb oder ein Direktantrieb, vorzugsweise mittels eines Elektromotors. Insbesondere ist es möglich, auch für jede Antriebswelle 104 bis 504 individuell jeweils einen Elektromotor vorzusehen.

## Patentansprüche

1. Vorrichtung zum Gären organischer Substanzen, insbesondere Gäroder Faulbehälter (1; 101), mit einem die organischen Substanzen bis zu einem Füllstand (2a) aufnehmenden Gärraum (2; 102), der eine im wesentlichen vertikal ausgerichtete Längsachse aufweist, und einer Mischeinrichtung, die antreibbare Mischmittel (3a bis 3g; 103; 203; 303; 403; 503) aufweist, wobei die Mischmittel (3a bis 3g; 103; 203; 303; 403; 503) mindestens teilweise über den Füllstand (2a) hinausragend aus den organischen Substanzen heraus bewegbar sind und anschließend wieder mindestens teilweise in die organischen Substanzen eintauchend bewegbar sind, so daß eine sich nahe oder an der Oberfläche vorhandene Schwimm- oder Deckschicht aufreißbar ist, **dadurch gekennzeichnet, daß** die Mischmittel (3a bis 3g; 103; 203; 303; 403; 503) auf einer Welle (4; 104; 204; 304; 404; 504) angeordnet sind, die um eine Achse rotierend antreibbar ist, und daß die Welle (4; 104; 204; 304; 404; 504) im wesentlichen horizontal ausgerichtet ist.

2. Vorrichtung nach Anspruch 1, **dadurch gekennzeichnet, daß** die Welle (4; 104; 204; 304; 404; 504) unterhalb des Füllstandes (2a) angeordnet ist.

3. Vorrichtung nach Anspruch 1 oder 2, **dadurch gekennzeichnet, daß** mehrere Mischmittel (3a bis 3g; 203; 303; 403; 503) entlang der Welle (4; 204; 304; 404; 504) hintereinander in einer Schraubenlinie angeordnet sind.

4. Vorrichtung nach einem der Ansprüche 1 bis 3, **dadurch gekennzeichnet, daß** mehrere Mischmittel (203; 303; 403) entlang der Welle (204; 304; 404) hintereinander alternierend diametral zur Achse angeordnet sind.

5. Vorrichtung nach einem der Ansprüche 1 bis 4, **dadurch gekennzeichnet, daß** die Mischmittel (3a bis 3g; 103; 203; 303; 403; 503) in ihrer Höhe in Bezug auf den Füllstand (2a) einstellbar ist.

6. Vorrichtung nach einem der Ansprüche 1 bis 5, **dadurch gekennzeichnet, daß** die Mischmittel (3a bis 3g; 103; 203; 303; 403; 503) flächig ausgebildet sind.

7. Vorrichtung nach Anspruch 6, **dadurch gekennzeichnet, daß** die Mischmittel (3a bis 3g; 203; 303) die Form eines Paddelblatts aufweisen und die von dem Paddelblatt ausgebildete Fläche eben ist und parallel zu der Achse ausgerichtet ist.

8. Vorrichtung nach Anspruch 6, **dadurch gekennzeichnet, daß** die Mischmittel (103; 403; 503) die Form eines schräggestellten Propellerblatts aufweisen und die von dem Propellerblatt ausgebildete Fläche mindestens zum Teil mit der Achse einen spitzen Winkel einschließt.

9. Vorrichtung nach einem der Ansprüche 1 bis 8, **dadurch gekennzeichnet, daß** die Vorrichtung mehrere Mischeinrichtungen aufweist, die nebeneinander angeordnet sind.

10. Vorrichtung nach einem der Ansprüche 1 bis 9, **dadurch gekennzeichnet, daß** die Vorrichtung weiterhin eine Schichtdickenmeßeinrichtung (14) aufweist zur Bestimmung der Dicke (15a) einer an der Oberfläche der Substanzen schwimmenden Deckschicht (15).

11. Vorrichtung nach einem der Ansprüche 1 bis 10, **dadurch gekennzeichnet, daß** die Vorrichtung weiterhin einen Gasraum (20) aufweist, der insbesondere durch eine in die Vorrichtung integrierte und eine aufblähbare Hülle (21) aufweisende Gashaube gebildet ist, und in dem ein beim Gären entstehender gasförmiger Energieträger auffangbar ist.

12. Verfahren zum Gären organischer Substanzen in einer Vorrichtung nach einem der Ansprüche 1 bis 11, **dadurch gekennzeichnet, daß** die Mischeinrichtung mindestens dann betrieben wird, wenn sich an der Oberfläche der organischen Substanzen im Gärraum (2; 102) eine den Aufstieg von Gasblasen behindernde Deckschicht (15) gebildet hat.

13. Verfahren nach Anspruch 12, **dadurch gekennzeichnet, daß** die Mischeinrichtung außerdem in von den Gärbedingungen vorgebbaren regelmäßigen Zeitabständen betrieben wird.

## Claims

1. An apparatus for the fermenting of organic substances, and especially a fermenting or decomposing container (1; 101), which incorporates a fermenting chamber (2; 102), which contains the organic substances up to a certain fill level (2a), whereby the same further incorporates a substantially vertical longitudinal axis, and a mixer, and whereby the same contains the mixing devices (3a to 3g; 103; 203; 303; 403; 503), whereby the mixing devices (3a to 3g; 103, 203; 303; 403; 503) moveably project at least in part over and above the fill level (2a) of the organic substances, and whereby subsequently at least a part of the same can be moveably submersed in the organic substances, so that a floating or covering layer near or upon the surface may be broken, **characterised in that** the mixing devices (3a to 3g; 103; 203; 303; 403; 503) are arranged in the shape of a shaft (4; 104; 204; 304; 404; 504) which is movably and rotatably arranged around an axis, and whereby the said shaft (4; 104; 204; 304; 404; 504) is substantially horizontal.

2. An apparatus according to Claim 1, **characterised in that** the shaft (4; 104; 204; 304; 404; 504) is located below the fill level (2a).

3. An apparatus according to Claim 1 or 2, **characterised in that** a multitude of mixing devices (3a to 3g; 203; 303; 403; 503) are positioned one after another along the shaft (4; 204; 304; 404; 504) in the form of a thread-like line.

4. An apparatus according to one of the preceding Claims 1 to 3, **characterised in that** a multitude of mixing devices (203; 303; 403) are positioned along the shaft (204; 304; 404) one after another in an alternating diametrical form in relation to the axis.

5. An apparatus according to one of the preceding Claims 1 to 4, **characterised in that** the height of the mixing devices (3a to 3g; 103; 203; 303; 403; 503) is adjustable in relation to the fill level (2a).

6. An apparatus according to one of the preceding Claims 1 to 5, **characterised in that** the mixing devices (3a to 3g; 103; 203; 303; 403; 503) are arranged in a surface-covering fashion.

7. An apparatus according to Claim 6, **characterised in that** the mixing devices (3a to 3g; 203; 303) are arranged in the form of a rudder leaf, and **in that** the area formed by the said rudder leaf is level and parallel in relation to the axis.

8. An apparatus according to Claim 6, **characterised in that** the mixing devices (103; 403; 503) are arranged in the form of a diagonally positioned propeller leaf, and **in that** the area formed by the said propeller leaf incorporates a sharp angle at least in a part of the same.

9. An apparatus according to one of the preceding Claims 1 to 8, **characterised in that** the said apparatus incorporates a multitude of mixers which are positioned adjacent to one another.

10. An apparatus according to one of the preceding Claims 1 to 9, **characterised in that** the said apparatus further incorporates a layer thickness measuring device (14) for the determination of the thickness (15a) of the covering layer (15) suspended upon the surface of the said substances.

11. An apparatus according to one of the preceding Claims 1 to 10, **characterised in that** the said apparatus further incorporates a gas chamber (20), which is formed substantially by a gas hood consisting of a sleeve (21) that is inflatably integrated into the apparatus, into which gaseous energy carriers created during the fermenting process can be collected.

12. A method for the fermenting of organic substances within an apparatus according to one of the preceding Claims 1 to 11, **characterised in that** the mixer is operated at least when a covering layer (15) is formed upon the surface of the organic substances contained within the fermenting chamber (2; 102) which prevents gas bubbles from rising upwards through the same.

13. A method according to Claim 12, **characterised in that** the mixer is further operated at regularly timed intervals which correspond to the relevant existing fermenting conditions.

## Revendications

1. Dispositif de fermentation de substances organiques, en particulier récipient de fermentation ou de putréfaction (1 ; 101), avec un compartiment de fermentation (2 ; 102), comprenant un axe longitudinal aligné essentiellement verticalement, recevant les substances organiques jusqu'à un niveau de remplissage (2a), et une installation de mélange qui comprend les moyens de mélange (3a à 3g ; 103 ; 203 ; 303 ; 403 ; 503) pouvant être commandés, moyennant quoi les moyens de mélange (3a à 3g ; 103 ; 203 ; 303 ; 403 ; 503) peuvent être déplacés au moins partiellement au dessus du niveau de remplissage (2a) en dehors des substances organiques et peuvent ensuite être à nouveau plongés au moins partiellement dans les substances organiques, de sorte qu'une couche surnageante ou de recouvrement située à proximité ou à la surface peut être désagrégée, **caractérisé en ce que** les moyens de mélange (3a à 3g ; 103 ; 203 ; 303 ; 403 ; 503) sont disposés sur un arbre (4 ; 104 ; 204 ; 304 ; 404 ; 504) pouvant être actionné de façon rotative autour d'un axe et **en ce que** l'arbre (4 ; 104 ; 204 ; 304 ; 404 ; 504) est aligné essentiellement horizontalement.

2. Dispositif selon la revendication 1, **caractérisé en ce que** l'arbre (4 ; 104 ; 204 ; 304 ; 404 ; 504) est situé sous le niveau de remplissage (2a).

3. Dispositif selon la revendication 1 ou 2, **caractérisé en ce que** plusieurs moyens de mélange (3a à 3g ; 103 ; 203 ; 303 ; 403 ; 503) se succèdent en hélice le long de l'arbre (4 ; 104 ; 204 ; 304 ; 404 ; 504).

4. Dispositif selon l'une des revendications 1 à 3, **caractérisé en ce que** plusieurs moyens de mélange (203 ; 303 ; 403) alternent successivement le long de l'arbre (204 ; 304 ; 404) diamétralement à l'axe.

5. Dispositif selon l'une des revendications 1 à 4, **caractérisé en ce que** les moyens de mélange (3a à 3g ; 103 ; 203 ; 303 ; 403 ; 503) sont réglables en hauteur par rapport au niveau de remplissage (2a).

6. Dispositif selon l'une des revendications 1 à 5, **caractérisé en ce que** les moyens de mélange (3a à 3g ; 103 ; 203 ; 303 ; 403 ; 503) sont configurés de façon plane.

7. Dispositif selon la revendication 6, **caractérisé en ce que** les moyens de mélange (3a à 3g ; 203 ; 303) présentent la forme d'une lame de palette et la surface formée par la lame de palette est plane et orientée parallèlement à l'axe.

8. Dispositif selon la revendication 6, **caractérisé en que** les moyens de mélange (103 ; 403 ; 503) présentent la forme d'une pale d'hélice biseautée, et la surface formée par la pale d'hélice forme un angle aigu au moins partiellement avec l'axe.

9. Dispositif selon l'une des revendications 1 à 8, **caractérisé en ce que** le dispositif comprend plusieurs installations de mélange placées les unes à côté des autres.

10. Dispositif selon l'une revendications 1 à 9, **caractérisé en ce que** le dispositif comprend en outre un système de mesure de l'épaisseur de couche (14) permettant de déterminer l'épaisseur (15a) d'une couche de recouvrement (15) surnageant à la surface des substances.

11. Dispositif selon l'une des revendications 1 à 10, **caractérisé en ce que** le dispositif comprend en outre un compartiment de gaz (20) qui est formé en particulier par un dôme de gaz intégré dans le dispositif et comprenant une enveloppe gonflable (21), et dans lequel un porteur d'énergie sous forme de gaz résultant de la fermentation peut être capté.

12. Procédé de fermentation de substances organiques dans un dispositif selon l'une des revendications 1 à 11, **caractérisé en ce que** l'installation de mélange est actionnée au moins dès qu'une couche de recouvrement (15) qui empêche l'ascension des bulles de gaz s'est formée à la surface des substances organiques dans le compartiment de fermentation (2 ; 102).

13. Procédé selon la revendication 12, **caractérisé en ce que** l'installation de mélange est en outre mise en marche à des intervalles de temps réguliers définis par les conditions de fermentations.
